# EUROPEAN PATENT APPLICATION

(11) **EP 2 308 551 A1**
(43) Date of publication of application: **13.04.2011**
(21) Application number: 10191017.2
(22) Date of filing: 08.12.2006
(51) Int. Cl.: A61N 1/05, A61B 17/34

(54) **System for blocking a nerve comprising an echogenic stimulating needle and a drug delivery catheter**

(30) Priority: 12.12.2005 US 749664 P
(62) Divisional of application: 06845014.7
(71) Applicant: Cook Critical Care Incorporated, Bloomington, IN 47404 (US)
(72) Inventor: Arndt, George, A., Madison, WI 53719 (US)
(74) Representative: Jehan, Robert

(57) **Abstract**

An apparatus and method for blockage of a peripheral nerve of a patient utilizes simultaneous continuous electrical nerve stimulation and visualization of the nerve using 2D ultrasound. A hyperechoic stimulating block needle is provided for insertion into the patient. The needle includes a hollow metal conduit, and a generally non-conductive covering extending along the shaft of the conduit. An echogenic surface capable of scattering and reflecting ultrasound waves for enhanced visualisation extends along at least a portion of the length of the needle. The needle is inserted into the patient, and the needle tip is optimally aligned in proximity with the nerve by simultaneous visualization with 2D ultrasound and by electrical nerve stimulation. Once the needle is optimally placed with regard to the nerve, a drug may be injected through a catheter passing through the needle, the catheter being capable of continuous administration of a drug therethrough upon removal of the needle.

## Description

### BACKGROUND

1. Technical Field. The present invention relates generally to a device for use in blocking a peripheral nerve of a patient to produce regional anesthesia, and more particularly, to a novel hyperechoic stimulating block needle suitable for such use. The invention further relates to a method for blocking a nerve utilizing simultaneous nerve stimulation and enhanced 2D ultrasound visualization.

2. Background Information. It is a well known medical practice to produce regional anesthesia in a patient by depositing a local anesthetic along the path of one or more peripheral nerves. The success of the technique is largely dependent upon the ability of the clinician to deposit a local anesthetic in close proximity to the nerve. Local anesthetics comprise a class of drugs which reversibly interact with a nerve in a manner such that the propagation of signals along the nerve fiber is significantly reduced, or stopped altogether. When such drugs are deposited along large nerve trunks, such as the femoral nerve in the groin or the nerve trunk of the brachial plexus in the axilla or neck, the effect is to make the targeted body structure, such as a body limb, insensate or "numb." This phenomena is similar to that experienced by a patient at a dentist's office when local anesthetics are used for placement of tooth fillings.

When the local anesthetic is intended to be injected into the groin, neck or axilla, the relevant nerve or nerves must, of course, be located before the injection is given. A general understanding of surface anatomy allows the general area of the nerve to initially be located. Historically the nerve was located by eliciting a paraesthesia, or pain, resulting from the needle coming into contact with the nerve fiber. This is very similar to the sensation experienced by hitting the "crazy bone", where the ulnar nerve is stimulated by pressure being placed on it between the skin and the bone. When this process is done with a needle, the risk of damaging a nerve fiber is high, with the possible result of permanent nerve injury. This technique has been largely abandoned due to the high possibility of such permanent injury.

The use of nerve blocks to accomplish such anesthesia has now progressed to the point where a stimulating needle may be utilized to locate a nerve, without making direct contact with the nerve. Nerve block systems are provided with certain features to minimize damage to the nerve. A first feature is to cut the needle end with a "B" bevel, at an angle of roughly 45 degrees. This action produces a lowered incidence of impinging nerve fibers when the needle is directed at a nerve trunk. This needle angle tends to allow nerve fibers to roll out of the way, as opposed to more common sharp tips seen in needles of the type that are used to puncture skin for the introduction of, e.g., solutions or catheters below the skin. A second feature is to provide the needle with a coaxial design, consisting of a needle shaft covered with a plastic coating, such as PTFE. The needle shaft is connected to an electrode, and the needle electrode system and a grounding skin electrode are connected to a commercially available nerve stimulation box.

An electrical circuit is formed when the needle is placed in the patient's tissue and the grounding electrode is connected to the patient's skin, e.g., with a conventional EKG electrode. The nerve stimulation resulting from this circuit is capable of delivering adjustable pulses of electrical energy through the needle. When the needle tip is in close proximity to the nerve, the motor nerve fibers are stimulated to cause muscles innervated by the nerve to twitch by electrical stimulation resulting from the electrical current flow in the electrical circuit. This mechanism is similar to that observed in a high school biology experiment, when the leg of a freshly dead frog is made to twitch by direct electrical stimulation of the nerve, thereby innervating the leg. In this nerve stimulation technique, the clinicians are, in effect, attempting to localize the nerve without actually puncturing the nerve tissue. This technique is intended to allow the needle to come close to the nerve, without actually contacting the nerve fibers in a manner that might cause permanent damage to the nerve.

Needle insertion by the aforementioned technique is based upon clinical judgment, and therefore, is not precise. The amount of electric current necessary to make the correct muscle twitch for the nerve to be blocked is determined by the proximity of the needle to the nerve. Generally, only a small amount of current is required, since resistance is typically minimal as the needle approaches the nerve. In clinical practice, this is typically performed at 2 to 4 Hz stimulation frequency, with an optimal current of 0.5 milliamps or less to bring the needle in close enough proximity to the nerve for drug injection. The actual voltage required is proprietary, and is a property of the particular peripheral nerve stimulator utilized in the technique. It is set at a value to produce a motor response without pain. A limitation of this technique is that it is a blind technique that is carried out based on a general understanding of the surface anatomy of the particular nerve to be blocked, and without a precise location of the nerve under the skin.

Ultrasound energy comprises high frequency sound waves generated in the 2 to 15 MHz range. In common medical practice, a range of 5 to 12 MHz is employed for most applications, as this range provides optimal tissue resolution and penetration. The sound waves are commonly generated using a piezoelectric crystal. Piezoelectric crystals produce ultrasound energy when electrically stimulated, and also respond to reflected ultrasound energy. The ultrasound energy is pulsed and time locked. Ultrasound energy is typically reflected, and this reflected ultrasound energy is capable of amplification. Measuring reflected amplified energy enables the clinician to determine a range or distance to a tissue interface. Medical ultrasound techniques, such as 2D medical ultrasound, typically employ a piezoelectric effect reflective head, a computer, an electronic component, and a monitor to display the anatomy generated by the ultrasound integration of the tissue being examined.

A 2D ultrasound technique typically uses an ultrasound head with a set of piezoelectric crystals in alignment, which crystals can be electronically switched on or off to respond to reflected ultrasound energy. The time delay between ultrasound emission and reflection can be used to construct a 2D picture of the tissue in alignment in the ultrasound plane generated. When the piezoelectric crystals are switched on and off electronically, a planar picture of the anatomy is created and displayed on the 2D ultrasound monitor. The 2D ultrasound machine allows tissue and anatomy to be visualized in both the axial and lateral direction. By controlling the switching order and timing of the individual piezoelectric crystals in the ultrasound head, the tissue can be scanned in a temporal fashion, thus creating a real time display of the tissue, and thus motion.

Ultrasound techniques, such as 2D ultrasound, are widely used in modern medicine. Such techniques are currently used for peripheral nerve blockage by allowing the clinician to view the nerve to be blocked in real time. In using a 2D ultrasound machine to block a nerve, the clinician is able to see below the skin, and thereby view the location of the nerves to be blocked. This renders greater precision in the procedure, and allows the clinician to advance the needle to the desired position relative to the nerve. A local anesthetic can then be deposited near the nerve to be blocked.

Conventional nerve stimulating block needles used in 2D ultrasound techniques are typically of coaxial design. These needles have an inner needle portion made of metallic material, typically surgical grade steel. A plastic matrix covers most of the length of the needle, and extends generally from the proximal end of the needle nearly to the bare metal needle tip. This type of needle construction ensures maximal current density, as the current can only exit at the unencased metal needle tip. The plastic covering of the needle insulates the remaining portion of the needle from the remaining patient tissue, ensuring that electrical current primarily exits at the needle tip. The needle tip, when in close proximity to the nerve, localizes the nerve with electrical stimulation while minimizing nerve damage.

One major shortcoming of the use of the conventional coaxial needle in a 2D ultrasound technique is that the needle is often not easily visible in the plane of the 2D ultrasound beam. Maximum reflection of ultrasound energy occurs when the needle is at a 90° angle to the direction of the ultrasound waves in the 2D ultrasound plane. The signal degrades as this angle is reduced, to a point at which the needle becomes invisible in the 2D ultrasound plane. This effect makes use of a coaxial stimulation needle problematic, since it is often ergonomically difficult to align it in the ultrasound head, define the tissue anatomy, and advance the needle in a 3D structure, while keeping the needle in view on the narrow 2D ultrasound plane.

It would be desirable to provide a stimulating needle for use in a 2D ultrasound technique having enhanced echogenicity when compared to existing needles, and with less signal degradation than experienced with the use of the conventional coaxial needle. In addition, it would be desirable to provide an improved mechanism for depositing a drug near a peripheral nerve by combining the respective advantages of electrical nerve stimulation and 2D ultrasound visualization.

### BRIEF SUMMARY

The present invention addresses the limitations of the prior art. A stimulating needle having an echogenic surface suitable for use in 2D ultrasound is provided. The echogenic surface enhances needle visualization by improving the reflectance of the ultrasound waves back to the ultrasound head.

A stimulating needle is formed by introducing irregularities, such as micro-scale deformations, along an axial surface of the needle. The presence of the irregularities improves the echogenic capacity of the needle by improving the ability of the needle to reflect ultrasound energy back to the ultrasound head.

In one form, the needle is constructed to have three separate components. The first component is a metal needle shaft that may be electrically connected to an electrode in conventional fashion. The needle preferably has a beveled tip, such as a well-known "B" bevel. The second component is a plastic coaxial covering material, such as PTFE. The plastic material preferably encases much of the length of the metal needle, but does not extend to cover the metal needle tip. The third component is an axial needle covering that is positioned over the PTFE plastic coating to substantially encase the needle. This covering, which may be metallic, would have the two properties noted. First, it has an irregular surface to improve the reflection of ultrasound energy to the ultrasound head. Second, it is electrically isolated from the metal matrix of the needle, so as to not compromise the electrical isolation of the tip of the needle, and to ensure maximal current density at the needle tip. As an additional or an alterative feature, the needle metal matrix may incorporate an echogenic surface under insulating the plastic material to achieve a similar result.

Thus, one feature of the present invention comprises a novel hyperechoic stimulating block needle. The use of this needle allows simultaneous ultrasonic visualization of peripheral nerves, and continuous electrical nerve stimulation while the needle is advanced toward the nerve under ultrasonic visualization. The hyperechoic stimulating block needle is electrically connected to a peripheral nerve stimulator. An electrical circuit with a grounding electrode and nerve stimulator can locate the position of peripheral nerves by nerve stimulation. The hyperechoic stimulating block needle may also be used to introduce catheters for long-term continuous infusion of drugs.

Another feature comprises a method for blockage of a nerve of a patient, utilizing ultrasound visualization and continuous nerve stimulation. A hyperechoic stimulating block needle is provided. The needle includes an electrically conductive needle conduit having a shaft portion and a distal tip portion. A generally non-conductive covering extends along the shaft portion of the conduit, and an echogenic material extends along at least a portion of the non-conductive covering. The needle tip is inserted into a patient and aligned in proximity with the nerve. An anesthetic may then be injected by simultaneous visualization of the nerve by ultrasound visualization, and stimulation of the nerve by electrical nerve stimulation.

### BRIEF DESCRIPTION OF THE DRAWINGS

Fig. 1 illustrates a perspective view of an inventive hyperechoic stimulating block needle according to one embodiment of the present invention;

Fig. 2 illustrates an enlarged view of an embodiment of a tip member for a hyperechoic stimulating block needle;

Fig. 3 is a diagram illustrating a hyperechoic stimulating block needle inserted into a patient and approaching a peripheral nerve, using a 2D ultrasound machine and peripheral nerve stimulation;

Figs. 4 and 4A are schematic diagrams illustrating the interaction between ultrasound waves and a coaxial electrical stimulating peripheral nerve block needle of the prior art; and

Fig. 5 is a schematic diagram illustrating the interaction between ultrasound waves and a hyperechoic nerve block needle according to an embodiment of the present invention.

### DETAILED DESCRIPTION OF THE DRAWINGS AND THE PRESENTLY PREFERRED EMBODIMENTS

For the purposes of promoting an understanding of the principles of the invention, reference will now be made to the embodiments illustrated in the drawings, and specific language will be used to describe the same. It should nevertheless be understood that no limitation of the scope of the invention is thereby intended, such alterations and further modifications in the illustrated device, and such further applications of the principles of the invention as illustrated therein being contemplated as would normally occur to one skilled in the art to which the invention relates.

In the following discussion, the terms "proximal" and "distal" will be used to describe the opposing axial ends of the stimulating block needle of the present invention, as well as the axial ends of various components thereof. The term "proximal" is used in its conventional sense to refer to the end of the needle (or component thereof) that is closest to the operator during use of the needle. The term "distal" is used in its conventional sense to refer to the end of the needle (or component thereof) that is initially inserted into the patient, or that is closest to the patient during use.

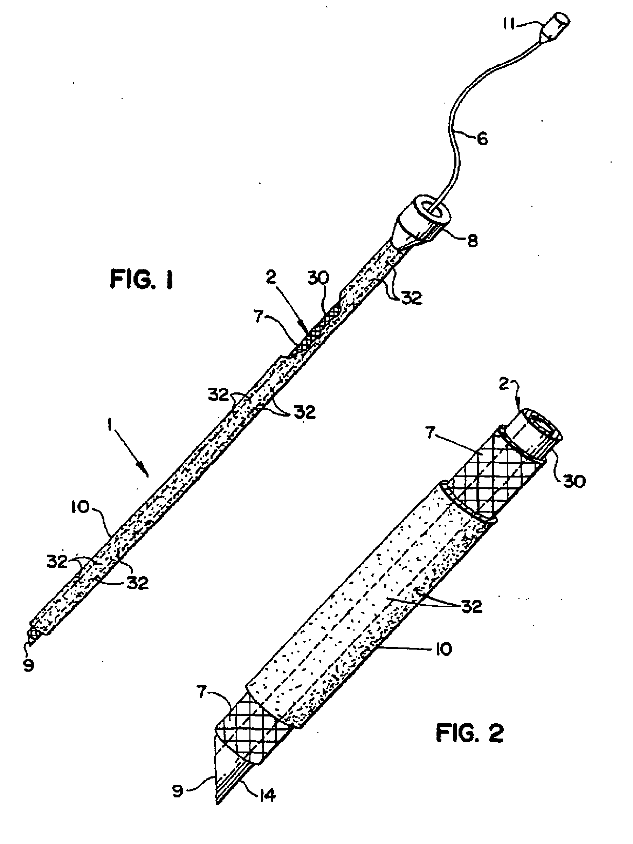

Fig. 1 illustrates a hyperechoic stimulating block needle 1 according to one embodiment of the present invention. Among other uses, needle 1 may be utilized in a process for blocking a Peripheral nerve that combines nerve stimulation and 2D ultrasound visualization techniques. Needle 1 is provided with an irregular surface that enhances the reflection of ultrasound waves toward an emitter receiver array of a 2D ultrasound machine, thereby allowing enhanced needle visualization with a 2D ultrasound machine. This allows the needle to be inserted into the patient's tissue and advanced toward a peripheral nerve with greater precision than may be achieved with either ultrasound or peripheral nerve stimulation alone. The technique may be utilized to deposit drugs near a nerve, e.g., to produce local or regional anesthesia at a targeted area in the body of the patient.

In the embodiment shown in Fig. 1, needle 1 includes a hollow elongated conduit 2. Conduits for use in stimulating needles are well known in the art, and conduit 2 may have a composition typically utilized for such purpose. Preferably, conduit 2 comprises an elongated shaft portion 30 formed of an electrically conductive metal or metal alloy, such as surgical grade steel, and an electrically conductive distal tip portion 9. Distal tip portion 9 may be formed of the same metal or alloy used for forming shaft 30, or from a different conductive material.

Tip 9 has sufficient sharpness to enable the needle to puncture the patient's skin, and advance through tissue. Preferably, tip 9 is an arcuate or beveled tip, and more preferably, is a short bevel tip. Although the short bevel tip is not restricted to a particular bevel angle, in a preferred embodiment, the bevel angle is about 45°. Those skilled in the art will appreciate that beveled tips at angles other than 45° (such as less than 45°) may be preferred in certain circumstances, and such tips are also within the scope of the invention. Typically, short bevel tips require more force during insertion than long bevels. However, this additional exertion of force enables the clinician to better "feel" the texture of the tissue as the tip is advanced, thereby helping to identify the tip location. Those skilled in the art are very familiar with various needle tips, and are suitably equipped to select a satisfactory tip for a particular application in view of the teachings of the present invention.

An electrically non-conducting insulating layer 7 covers at least a portion of conduit 2. Preferably, insulating layer 7 covers substantially the entire length of conduit shaft portion 30, but does not cover tip portion 9. Providing the insulating covering on shaft portion 30 insulates the shaft from the remaining patient tissue, and ensures that maximal electrical current exits at tip portion 9. Preferably, the covering material is a plastic, such as PTFE. Although insulating layer 7 need not necessarily extend along the entire length of shaft 30, this arrangement is preferred so that maximum current density is provided at the tip.

In the preferred embodiment shown, at least a portion of the electrically non-conducting layer 7 is covered by an echogenic encasing sheath 10. Sheath 10 may comprise a conventional jacket or tube, or may alternatively comprise a coating layer that covers all or part of layer 7. The sheath is preferably formed of a metal or metal alloy, and is provided with an irregular surface. In the embodiment shown, the irregular surface comprises a plurality of deformations 32 distributed along the exterior surface of sheath 10. Deformations 32 are imperfections that are formed along the sheath surface in a manner that enhances the ability of the needle to scatter and/or reflect ultrasound energy back to the ultrasound head, thereby improving the echogenic capacity of the needle. The deformations may be formed along the length of the sheath by well-known processes, such as sandblasting, physical deformation, micro hammering, etc. Those skilled in the art will appreciate that there are many other ways of forming surface deformations of a type that will result in the scatter and/or reflectance of ultrasound waves that may be substituted for the techniques described above. Deformations 32 do not adversely affect the mechanical properties of the needle with respect in the ability of the needle to pass through tissue.

The presence of the imperfections, such as deformations 32, causes ultrasound waves that contact the deformations to travel in multiple directions and in a more random fashion than with conventional needles. The increase in scatter and/or reflection of the ultrasound waves enhances the temporal visualization of the hyperechoic needle path and tip during 2D ultrasound examination. This action significantly improves the axial, lateral and temporal resolution of the stimulating needle under 2D ultrasound. In practice, the deformations enhance the visibility of the needle under 2D ultrasound, regardless of the needle orientation to the ultrasound head. The needle will appear on the 2D ultrasound monitor in real time, and the needle position, needle path tip, and interaction with the tissue will be visible in real time. As a result, with a visible echogenic needle, the needle may be safely advanced toward the nerve using the 2D ultrasound monitor.

Although the echogenic sheath has been described herein as a jacket or a coating applied over the insulating layer of the needle, this arrangement is not required. In an alternative embodiment, the echogenic surface may comprise the deformation of a surface, such as a cannula or the shaft of the needle, that is positioned under the insulating layer. In this event, the ultrasound beam passes through the insulating layer, and reflects off the cannula or shaft surface back to the ultrasound head. As a still further alternative, the echogenic surface may result from the combined effect of deformation over and under the insulating layer.

The echogenic layer need not be continuous along the length of the needle. Rather, the echogenic layer may be discontinuous along the needle axis, and the length of the needle may include discrete lengths having, and not having, irregularities of deformities. This arrangement provides additional contrast along the needle surface, thereby allowing the clinician to delineate position, path, and length of the needle using 2D ultrasound. Similarly, the echogenic layer need not be structured to provide only a single type of echogenic signal. Rather, the layer may be structured with more than one type of imperfection or deformity, to provide different types of echogenic signals along the needle axis, thereby providing additional contrast and/or visibility along the needle surface.

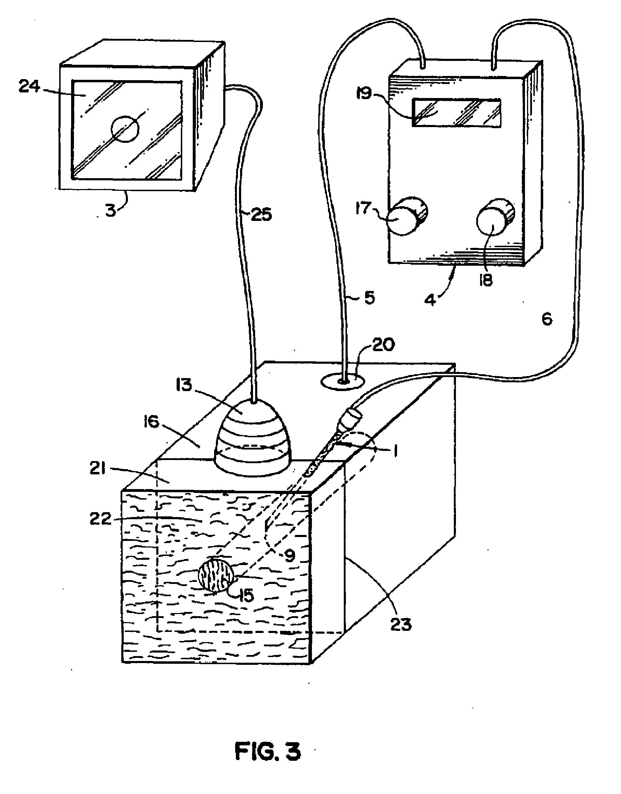

In the embodiment shown, a generally tubular metal or plastic hub 8 is engaged to the proximal end of shaft 30. When present, hub 8 is sized and shaped for attachment to a syringe, tube, or other medical device in well known fashion. Echogenic encasing sheath 10 is preferably electrically isolated from the tubular hub 8 and hollow metal conduit 2. To utilize needle 1 in a circuit, an electrode 6 may be electrically connected at one end to the metal conduit 2 or hub 8. and at the other end to a conventional peripheral nerve stimulator 4 (Fig. 3). The proximal end of electrode 6 may terminate at a mechanical connector 11 of a type that is suitable for connection to an outlet of the peripheral nerve stimulator 4.

Fig. 2 is an enlarged view of the distal end or tip 9 of the hyperechoic stimulating block needle 1 of Fig. 1. In the embodiment shown, needle tip 9 is bevel cut at an angle of approximately 45°, or in other words, at a less acute angle than a standard needle. The unencased echogenic needle tip portion 14 may also be rendered echogenic by deforming the surface extending from the needle end 9 to the insulating coating 7. As a result, the needle tip can be distinguished from the remaining hyperechoic stimulating block needle 1 as viewed by 2D ultrasound.

Fig. 3 illustrates a system for peripheral nerve block. The system includes a hyperechoic stimulating block needle 1, a medical imaging mechanism 3, and a peripheral nerve stimulator 4. Preferably, the imaging mechanism comprises an ultrasound machine, and more preferably, a 2D ultrasound machine. Those skilled in the art will recognize that other medical imaging mechanisms capable of receiving an array of detectable beams may be substituted for the 2D ultrasound mechanism described in the preferred embodiment herein. Peripheral nerves stimulation devices are known in the art, and a skilled artisan can readily select an appropriate device in view of the teachings herein.

A peripheral nerve 15 of the patient is depicted in Fig. 3 in a block of tissue 16. The remaining portions of the patient's anatomy are not relevant to gaining an understanding of the system, and are therefore not shown in the figure. In the embodiment shown, peripheral nerve stimulator 4 has two controls, namely a frequency control knob 17, and an amperage or current control knob 18. Also, in the example shown, peripheral nerve stimulator 4 is provided with an optional digital readout 19 for displaying the current when a circuit is formed.

A circuit is formed by attaching a grounding electrode 5 that extends from the peripheral nerve stimulator 4 to a conventional electrode 20 of the type that is placed on the patient's skin 21, and a needle electrode 6 that extends from the hyperechoic stimulating block needle 1 to the peripheral nerve stimulator 4. The hyperechoic stimulating block needle 1 is inserted into patient tissue 22. A pathway is thereby formed for electrons to flow from peripheral nerve stimulator 4 through the needle electrode 6 to the hyperechoic stimulating block needle 1, and through the shaft 30 (which is electrically insulated from the patient) and the tip 9 of the needle. The electrons pass through the patient's tissue 22 and exit the patient through skin electrode 20, returning to the peripheral nerve stimulator 4 via grounding electrode 5. Peripheral nerve 15 is located by activating the peripheral nerve stimulator 4 to form the circuit. Stimulator frequency knob 17 is adjusted to emit an electrical pulse, most commonly with a range of 2 to 4 Hz. Stimulator amperage control knob 18 is adjusted to elicit a motor response when the needle is advanced in the region of the peripheral nerve 15. The amperage is commonly set at about 2 milliamps to search for the general nerve location.

The needle is advanced toward the peripheral nerve 15 using general knowledge of surface anatomy, and with the guidance of the 2D ultrasound machine 3. The 2D ultrasound machine 3, shown schematically in Fig. 3, typically comprises a monitor 24, a computer (not shown), a cable or head cord 25 and an ultrasonic head 13. Ultrasonic head 13 typically includes a series of piezoelectric effect crystals in alignment. The ultrasound head 13 is capable of sending out a series of ultrasound beams, and to receive reflected energy. The reflected energy is amplified, processed, and integrated in 2D ultrasound machine 3, thereby rendering a 2D planar image of the tissue below the head.

The nerve 15 is rendered visible in a lateral and axial fashion in the plane of the 2D ultrasound machine 23, and displaced as a planar 2D image on the monitor 24 of the 2D ultrasound machine 3. The signal from the ultrasound machine head 13 is received by the 2D ultrasound machine 3 via the 2D ultrasound head cord 25. The peripheral nerve stimulator 4 emits a square wave DC current at a predetermined voltage (e.g., typically about several hundred volts) that is determined according to the characteristics of the peripheral nerve stimulator. As the hyperechoic stimulating block needle 1 is advanced toward the peripheral nerve 15, the motor response is elicited by stimulation of the motor nerve fibers by current flowing through the nerve. The correct nerve to be blocked can be determined by a general understanding of the anatomy of the nervous system, and in particular, by recognizing which nerve will cause a specific part of the body to move as a result of the electrical stimulation.

The motor response is different for each nerve to be blocked. As the needle is advanced toward the target nerve, the motor response becomes more intense, since less tissue is present between the needle tip 9 and peripheral nerve 15, thereby reducing the resistance to current flow. The current is decreased as the needle approaches the nerve, as less current is required to elicit a motor response. Clinically when the current is at 0.5 milliamps or less, the needle tip 9 is in close enough proximity to the nerve that a local anesthetic can be injected, thereby achieving the desired clinical response of making the area innervated by the nerve 15 insensitive or numb.

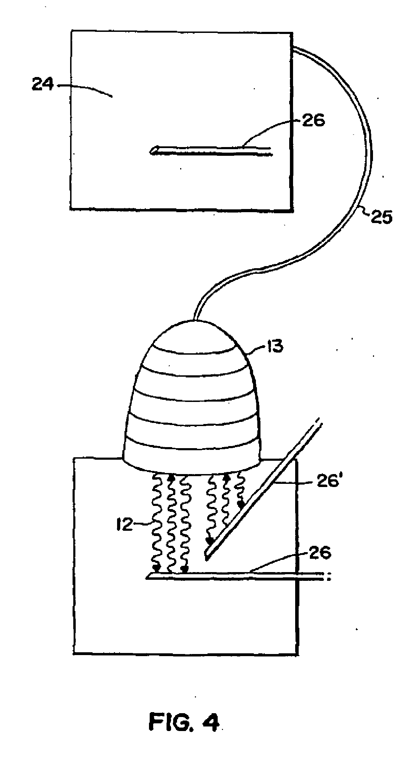

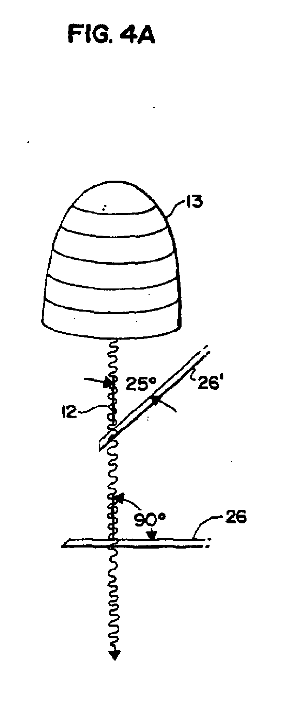

Fig. 4 depicts a display on a 2D ultrasound monitor of a conventional coaxial stimulating block needle 26 in the 2D ultrasound plane 23 in a tissue block. Fig. 4A depicts a display substantially similar to that of Fig. 4, but indicating reference points to various angles cited herein. The coaxial stimulating block needle 26 is seen optimally on monitor 24 when it is oriented at a 90° angle to the ultrasound beam. However, the ability to resolve the coaxial needle image on the 2D ultrasound monitor 24 degrades as the needle moves from the 90° orientation to a lesser orientation, at which point it becomes invisible. Fig. 4A provides a frame of reference for the angles specified. Note in the figure that angled needle 26' is invisible on ultrasound monitor 24. In the example shown, the orientation of the needle 26 has been rotated 65° from the 90° orientation to an angle of 25°. This phenomenon is caused by specular reflectance, as the plastic coating will only reflect ultrasound waves 12 directly back to the ultrasound head 13. This reflectance is generally similar to the way that light is reflected from a mirror. The specular reflectance of the coaxial block needle 26 makes needle visualization with 2D ultrasound difficult. Commonly, the needle is invisible as it is advanced, greatly decreasing the utility of 2D ultrasound by allowing only peripheral nerve stimulation to resolve needle tip position, resulting from decreasing amperage requirement to elicit a motor response.

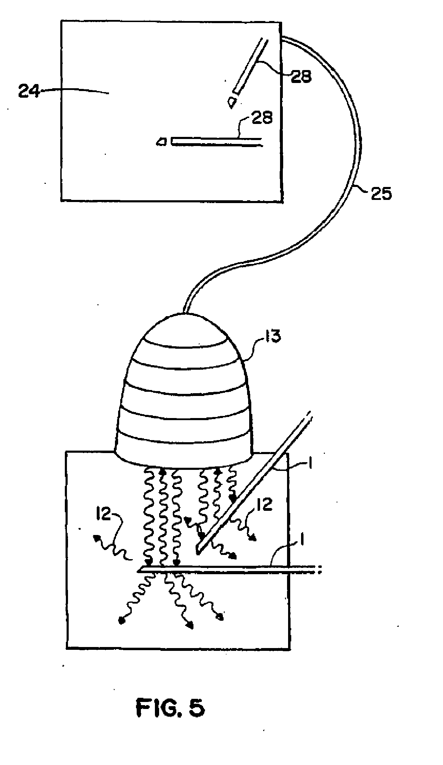

Fig. 5 depicts a display on a 2D ultrasound monitor 24 of a hyperechoic stimulating block needle 1 according to the present invention in a tissue block. The hyperechoic stimulating block needle 1 has an echogenic layer 10 as described hereinabove, which echogenic layer is structured to maximally reflect ultrasound waves 12 back to the ultrasound head 13. This renders the hyperechoic stimulating block needle visible 1 as it moves from a 90° orientation (e.g., perpendicular) to the direction of the ultrasound waves to an orientation that approaches an alignment with the ultrasound waves. The hyperechoic stimulating block needle image 28 is thus visible in the 2D ultrasound plane 23 with greater resolution than may be achieved with the coaxial needle 26, both at the 90° angle, and at lesser angles. In Fig. 5, needle image 28 is visible after the needle has rotated 65° from the perpendicular orientation described.

Echogenic layer 10 enables the hyperechoic stimulating needle 1 to be seen as it is advanced in the 2D ultrasound plane 23, rendering both the needle path and needle tip 9 visible as the needle approaches the peripheral nerve 15. This reflection is different than specular reflectance, since it results from the scattering of the ultrasound waves 12 by the echogenic layer 10. In this case, wave scattering occurs toward the 2D ultrasound head 13 to make the hyperechoic stimulating block needle image 28 visible at various angles in the 2D ultrasound plane 23.

The ability to see the needle path, needle tip 9, and the peripheral nerve 15 to be blocked, in combination with peripheral nerve stimulation, allows the peripheral nerve 15 to be approached with more precision than has previously been possible. The position of the needle can be resolved anatomically using 2D ultrasound by simultaneous visibility of the needle path, needle tip 9 and peripheral nerve 15, and can be resolved physiologically by using peripheral nerve stimulation to elicit a motor response by minimizing the current. This allows the needle tip 9 to be directed to a closer proximity to the peripheral nerve 15 when compared to the use of 2D ultrasound, peripheral nerve stimulation and a coaxial peripheral stimulating nerve block needle, as shown in Fig. 4. As a result, drugs can be deposited more precisely than has previously been possible using either ultrasonic visualization or peripheral nerve stimulation separately.

The use of hyperechoic stimulating block needle 1 also provides a mechanism for passing a catheter through the needle using 2D ultrasound, and for removing the needle leaving the catheter in place for continuous administration of drugs. Similarly, the catheter may have an echogenic material incorporated in the catheter matrix, or at the tip of the catheter matrix, to enable visualization and advancement of the catheter through the needle, and correct anatomical position using ultrasound. Still further, a catheter may incorporate in the catheter matrix a metal or electrically conductive echogenic material to allow an electrical circuit to be formed with a peripheral nerve stimulator, by utilizing an electrode running the catheter matrix from the distal to proximal end. Alternatively, the catheter can be fully or partially filled with an electrically conductive material such, as a saline solution.

It is therefore intended that the foregoing detailed description be regarded as illustrative rather than limiting, and that it be understood that it is the following claims, including all equivalents, that are intended to define the spirit and scope of this invention.

In one embodiment there is provided a hyperechoic stimulating block needle for use in blocking a nerve of a patient using simultaneous electrical nerve stimulation and 2D ultrasound visualization of the nerve, the needle comprising:
a hollow needle conduit, said conduit comprising a shaft portion and a tip portion,
an insulating layer covering at least a portion said needle shaft portion, and
an echogenic surface disposed along a length of said needle, said echogenic surface adapted for enhancing a reflection of ultrasound waves generated during said 2D ultrasound visualization.

Preferably, said echogenic surface comprises an outer layer covering at least a portion of said insulating layer.

Preferably, said hollow needle conduit is structured such that said conduit is electrically conductive upon electrical connection to a conducting electrode, said insulating layer substantially covers said conduit shaft portion and not said tip portion, and said outer layer is positioned such that said outer layer is substantially electrically isolated from said conduit.

Preferably, said insulating layer comprises a substantially non-conductive component, and said echogenic surface comprises a metal or metal alloy.

Preferably, said substantially non-conductive component comprises PTFE, and said echogenic surface comprises surgical grade steel.

Preferably, said tip comprises a bevel.

Preferably, said bevel tip has an angle not exceeding about 45°.

Preferably, said echogenic surface comprises a plurality of deformations disposed along at least a portion of a length of said outer layer, said deformations shaped and positioned to enhance said ultrasound wave reflection.

Preferably, said echogenic surface is disposed interiorly of said insulating layer.

In one embodiment there is provided a method for blockage of a nerve of a patient, comprising:
providing a hyperechoic stimulating block needle, said needle comprising an electrically conductive hollow needle conduit, said conduit having a shaft portion and a distal tip, a generally non-conductive covering extending along said conduit shaft portion, and an echogenic surface extending along at least a portion of a length of said needle;
inserting said needle tip into a patient;
aligning said needle tip in proximity with said nerve by visualization with medical imaging and by electrical nerve stimulation; and
injecting a drug through a bore of said hollow needle conduit into said patient. Preferably, said visualization comprises 2D ultrasound.

Preferably, said electrical nerve stimulation comprises emitting an electrical pulse in a vicinity of said nerve, and adjusting an amount of current to elicit a motor response when said needle tip is advanced toward said nerve.

Preferably, said electrical pulse is emitted at about 2 to 4 Hz, and said current is adjusted at about 2 milliamps.

Preferably, said current is reduced as said needle tip approaches said nerve to an amount not exceeding about 0.5 milliamps prior to injection of said drug.

In one embodiment, there is provided a system for use in blocking a nerve of a patient, comprising:
a hyperechoic stimulating block needle, said needle comprising an electrically conductive needle conduit, said conduit having a shaft portion and a distal tip, a generally non-conductive covering extending along said conduit shaft portion, and an echogenic material extending along at least a portion of a length of said needle;
a peripheral nerve stimulator capable of electrical connection with the needle for submitting an electrical pulse therethrough; and
a medical imaging mechanism capable of visualizing at least a portion of the needle in a segment of tissue of the patient.

Preferably, said medical imaging mechanism comprises 2D ultrasound.

Preferably, said peripheral nerve stimulator is capable of providing an adjustable current for eliciting a motor response.

Preferably, said echogenic material comprises an outer layer extending over at least a portion of said generally non-conductive covering, wherein said outer comprises a plurality of deformations disposed along at least a portion of a length of said outer layer, said deformations shaped and positioned to enhance reflection of ultrasound waves generated by said imaging mechanism.

Preferably, said non-conductive coating comprises a polymeric composition, and said echogenic surface comprises a metal or metal alloy.

Preferably, said echogenic material is disposed interiorly of said non-conductive coating.

The disclosures in European patent application 06845014.7 (published as WO 2007/070374), from which this application is divided, and the abstract accompanying this application, are incorporated herein by reference.

## Claims

1. A hyperechoic stimulating block needle assembly for blocking a nerve of a patient using simultaneous electrical nerve stimulation and 2D ultrasound visualization of the nerve, comprising a needle, the needle comprising:
a hollow needle conduit, said conduit comprising a shaft portion and a tip portion,
an insulating layer covering at least a portion of said needle shaft portion, and
an echogenic surface disposed along a length of said needle, said echogenic surface adapted for enhancing a reflection of ultrasound waves generated during said 2D ultrasound visualization.

2. The needle assembly of claim 1, comprising:
a catheter passing through the needle, said catheter capable of continuous administration of a drug therethrough upon removal of the needle.

3. The needle assembly of claim 2 wherein the needle is operable to have an electrical pulse submitted through it for electrical nerve stimulation and wherein the echogenic surface comprises irregularities.

4. The needle assembly of claim 2 or 3, wherein the catheter comprises an echogenic material to enable ultrasound visualization of the catheter.

5. The needle assembly of claim 4, wherein the echogenic material is incorporated into the catheter matrix or is at the tip of the catheter matrix.

6. The needle assembly of any of claims 2 to 5, wherein the echogenic surface disposed along the length of the needle comprises an outer layer covering at least a portion of said insulating layer of said needle.

7. The needle assembly of claim 6, wherein said hollow needle conduit is structured such that said conduit is electrically conductive upon electrical connection to a conducting electrode, said insulating layer substantially covers said conduit shaft portion and not said tip portion, and said outer layer is positioned such that said outer layer is substantially electrically isolated from said conduit.

8. The needle assembly of any of claims 2 to 7, wherein the insulating layer comprises a substantially non-conductive component, and said echogenic surface comprises a metal or metal alloy.

9. The needle assembly of claim 6 or any of claims 7 to 8 when dependent on claim 6, wherein the echogenic surface comprises a plurality of deformations disposed along at least a portion of a length of the outer layer, said deformations configured and positioned to enhance said ultrasound wave reflection.

10. The needle assembly of claim 2 or 3, wherein said echogenic surface is disposed interiorly of said insulating layer.

11. The needle assembly of any of claims 2 to 10, wherein said echogenic surface is discontinuous along the needle axis.

12. The needle assembly of any of claims 2 to 11, wherein discrete lengths of the needle echogenic surface have irregularities, and other discrete lengths of said needle echogenic surface do not have irregularities.

13. The needle assembly of any of claims 2 to 12, wherein said needle echogenic surface comprises more than one type of irregularities, said irregularities configured and arranged along said echogenic surface in a manner to provide contrasting types of echogenic signals along the needle axis.

14. A system for use in the continuous administration of a drug for blocking a nerve of a patient, comprising:
a hyperechoic stimulating block needle, said needle comprising an electrically conductive needle conduit, said conduit having a shaft portion and a distal tip, a generally non-conductive covering extending along the conduit shaft portion, and an echogenic material extending along at least a portion of a length of the needle;
a catheter passing through the needle, said catheter capable of continuous administration of a drug therethrough upon a removal of the needle.
a peripheral nerve stimulator capable of electrical connection with the needle for submitting an electrical pulse therethrough; and
a 2D ultrasound imaging mechanism capable of visualizing at least a portion of the needle in a segment of tissue of the patient.

15. The system of claim 14, wherein said catheter has an echogenic material incorporated into the catheter matrix, or is at least partially filled with an electrically conductive material.

16. The system of claim 14 or 15, wherein said needle echogenic material comprises an outer layer extending over at least a portion of said generally non-conductive covering, said outer layer comprising a plurality of deformations disposed along at least a portion of a length of said outer layer, said deformations shaped and positioned to enhance reflection of ultrasound waves generated by said imaging mechanism.

17. The system of claim 14, 15, or 16, wherein discrete lengths of said needle echogenic material include irregularities for enhancing a reflection of ultrasound waves, and other discrete lengths of said needle echogenic surface do not include irregularities.
